# FASCICULE DE BREVET EUROPEEN

(11) **EP 0 377 343 B1**
(45) Date de publication et mention de la délivrance du brevet: **15.09.1993**
(21) Numéro de dépôt: 89400003.3
(22) Date de dépôt: 02.01.1989
(51) Int. Cl.: A61D 17/00, A01K 29/00

(54) **Dispositif de détection et d'alarme de parturition**
Alarmvorrichtung zum Melden des Geburtsbeginns
Parturition alarm apparatus

(43) Date de publication de la demande: 11.07.1990
(73) Titulaire: Menetrier, Jean-Paul, F-21350 Vitteaux (FR); Seignot, Jean-Yves, F-21390 Precy sous Thil (FR)
(72) Inventeur: Menetrier, Jean-Paul, F-21350 Vitteaux (FR); Seignot, Jean-Yves, F-21390 Precy sous Thil (FR)
(74) Mandataire: Bruder, Michel

(56) Documents cités:
- FR-A- 2 382 885
- FR-A- 2 392 599
- FR-A- 2 582 933

## Description

La présente invention concerne un dispositif pour la détection du vêlage imminent d'une vache et son signalement à un poste fixe de surveillance.

Chaque éleveur connaît bien les difficultés sinon les dangers encourus par les nouveaux-nés lors du vêlage d'une vache, qui, pratiquement, contraint le même éleveur à être présent et actif durant toute l'opération de mise bas. De ce fait, il devient essentiel pour l'éleveur de bien appréhender les indices ou les signes préliminaires annonçant à coup sûr un vêlage prochain de manière à en assurer le bon déroulement.

Ceci implique donc une surveillance sévère des bêtes arrivées à terme et ce de jour comme de nuit bien sûr. Il est par ailleurs courant d'effectuer des saillies en nombre, ce qui ajoute encore à la difficulté de surveillance dans la mesure où les vêlages vont se présenter dans la même période.

C'est pour toutes ces raisons que les éleveurs ont cherché par tous les moyens des indicateurs de vêlage leur permettant de ne pas être mobilisés indûment, voire tout simplement de ne pas rester en éveil constant, notamment la nuit. A cet égard de nombreux détecteurs ont été essayés, tels que les dispositifs mécaniques décrits par exemple dans les brevets FR-A-2 349 318 et FR-A-2 382 885 qui prennent en compte les déformations dues au travail des bêtes, parfois même sanglées dans ce but, pour déclencher des alarmes diverses. Ces dispositifs sont peu fiables car ils ne peuvent réellement différencier les déformations spécifiques au travail de vêlage, des déformations, parfois même fugitives, dues à la vie normale des bêtes.

D'autres détecteurs, comme celui qui est décrit dans le brevet FR-A-2 266 489, ont donné satisfaction en suivant la variation de certains paramètres caractéristiques du vagin des bêtes tels que température, pression, acidité ou déformations diverses. Pour cela, des sondes adaptées doivent être positionnées avec précision dans la vulve des bêtes, ce qui est un travail délicat, et leurs indications doivent être constamment analysées et interprétées. En outre, ces dispositifs coûteux sont largement parasités par la vie des bêtes dans leurs mouvements quotidiens, et constituent un vecteur de contagion pour certaines maladies.

Plus simplement enfin, certains éleveurs se contentent d'une surveillance télévisuelle des évolutions physiques caractéristiques d'un vêlage prochain, telles que l'ouverture du col, ou plus généralement d'autres indices caractéristiques comme par le redressement permanent de la queue de la vache au travail.

Cette solution, qui permet une surveillance à distance, particulièrement avantageuse la nuit, s'applique avec succès pour une surveillance en nombre par balayage de la caméra, malheureusement, elle contraint le surveillant à un éveil permanent et représente un investissement particulièrement onéreux.

La présente invention a pour but de remédier à de tels inconvénients dans la mesure où le dispositif proposé prend appui sur un indice objectif et fiable, précurseur de vêlage d'une vache, à savoir le relevage de sa queue de façon constante durant une période pouvant varier entre 4 minutes et 12 minutes avant la mise bas, tout en éliminant les événements de la vie quotidienne des bêtes parasitant la surveillance. En effet, le relevage de la queue pour une vache, en dehors de la période de pré-vêlage, est fréquent, car il constitue une nécessité de la vie des bêtes, notamment pour effecteur leurs besoins naturels. Etant observé que de tels relevages, parasites de la détection, ne sont que de courte durée, il est proposé, suivant l'invention, un dispositif pour le signalement du vêlage imminent d'une vache en détectant le relevage de la queue qui reste, de ce fait, en position redressée pendant une durée suffisante pour déclencher ledit dispositif. Ce dispositif comprend un détecteur de position angulaire temporisé déclenchant un système associé d'alarme autonome, caractérisé en ce que ledit détecteur de position angulaire est fixé autour de la queue au moyen d'un pince-queue ou de tout autre moyen de fixation équivalent et disposé de préférence juste à la naissance de ladite queue. De cette manière, le dispositif selon l'invention ne gêne en aucune façon les mouvements quotidiens et habituels de la vache. On peut ainsi l'utiliser sans problème en stabulation libre.

En outre, le dispositif peut-il être diversement sollicité suivant les mouvements habituels de la queue de la vache ainsi équipée, sans pour autant déclencher les organes d'alarme puisque le système temporisateur associé au détecteur de position angulaire n'autorisera leur déclenchement qu'après une station prolongée de la queue de la vache en position relevée. Ainsi les relevages fréquents et de courte durée de la queue pour uriner ou chasser les insectes par exemple, sont-ils effacés, procurant au dispositif toute sa fiabilité.

Dès que le détecteur de position angulaire, qui peut être un simple contacteur à mercure, à billes ou à lamelles, a été maintenu contact fermé continuellement pendant une durée suffisante présélectionnée d'environ 4 à 12 minutes, le temporisateur déclenche de façon irréversible un système d'alarme qui peut être lui-même fixé sur la vache et même intégré au dispositif de détection temporisé, ou, dans une variante avantageuse de l'invention, un émetteur miniaturisé alimenté classiquement de façon autonome, transmettant un signal à une station fixe ou mobile de surveillance qui, de ce fait, peut être relativement éloignée. Le signal de l'émetteur peut même être codé s'il s'agit de surveillance de plusieurs bêtes éventuellement éloignées les unes des autres.

Inversement, si le détecteur de position angulaire est sollicité pendant une durée inférieure à celle qui a été programmée sur la temporisation, dès que cesse ladite sollicitation ledit détecteur est réinitialisé immédiatement dans l'attente d'une nouvelle sollicitation sans aucun cumul des temps de relevages intempestifs de la queue de la bête à surveiller.

On décrira ci-après, à titre d'exemple non limitatif, une forme d'exécution de la présente invention, en référence au dessin annexé sur lequel :
- la figure 1 représente un dispositif détecteur de vêlage suivant l'invention, installé sur la queue d'une vache.
- la figure 2 est une vue en plan du dispositif montrant l'empilage des fonctions de détection, d'émission et d'alimentation.
- la figure 3 représente une variante du dispositif détecteur de vêlage suivant l'invention en position de signaler que le vêlage est prochain par une émission radio codée reçue par une station fixe de surveillance.

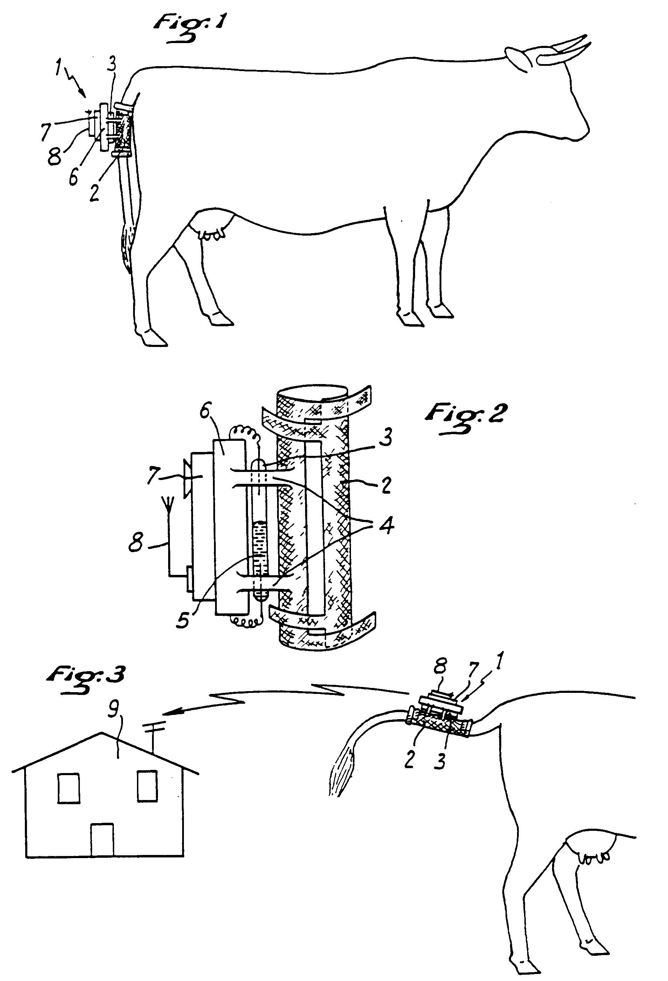

Conformément à la figure 1, le dispositif 1 détecteur de vêlage est fixé à la queue de la vache en gestation par un simple pince-queue 2, habituellement destiné à limiter l'amplitude des débattements de la queue des vaches pendant la traite. Le pince-queue 2 est constitué classiquement d'une lame ressort venant serrer de part et d'autre la queue de l'animal, et préservant de façon habituelle sa partie intérieure sensible. L'ensemble 1 est disposé juste à la naissance de la queue de façon à éviter, d'une part, les trop grands débattements qui risqueraient de le détériorer, et pour conserver, d'autre part, une verticalité suffisante du détecteur de position angulaire au repos, même lorsque la vache est en station allongée.

Le dispositif, suivant la forme d'exécution représentée sur la figure 2, comporte, outre le système de fixation par pince-queue 2, un détecteur à mercure 3 disposé le long du pince-queue 2, par l'intermédiaire de deux colliers 4. Le détecteur 3 est constitué classiquement d'un tube en une matière isolante et suffisamment résistante aux chocs extérieurs et intérieurs dus au déplacement du mercure 5. Deux électrodes sont disposées à chaque extrémité dudit tube, de telle manière qu'en position verticale aucun contact électrique ne puisse se faire entre les électrodes grâce au mercure 5 et qu'en position légèrement inclinée par rapport à l'horizontale, les deux électrodes soient mises en liaison électrique par le mercure 5 en fermant ainsi le circuit d'alimentation d'un relais temporisateur 6 dont le circuit de commande n'entre en action qu'après une certain période, réglable entre 4 et 12 minutes, d'alimentation continue, correspondant au critère retenu pour donner l'alerte d'un prochain vêlage de la vache.

Le circuit temporisateur 6, d'un type tout à fait connu en électronique, est muni d'un circuit de réinitialisation du temps de temporisation dès que cesse l'alimentation du relais par ouverture du contact des électrodes du détecteur à mercure 5, dont toute nouvelle sollicitation fait répartir un nouveau comptage.

Le système d'alarme 7 n'est, quant à lui, mis en action qu'après un comptage continu de temps préréglé.

Le système d'alarme 7 conforme à la figure 3 est un simple émetteur produisant un signal codé en fréquence transmis par une antenne 8 et dont la réception et le décodage à un poste fixe de surveillance 9 déclenchent l'alerte de l'éleveur qui peut alors agir rapidement pour les soins de la bête durant le vêlage. Il est clair que le poste de surveillance 9 peut être directement transporté en divers lieux, donnant à l'éleveur tout facilité de se déplacer. De même le poste fixe 9 peut être relayé par n'importe quel dispositif classique de transmission autonome et portable. Dans une autre variante du poste fixe 9 de surveillance, il peut être avantageusement complété par un dispositif d'alarme à retardement réglable après que le détecteur ait fonctionné, laissant au surveillant juste le temps qu'il juge nécessaire pour intervenir dans "l'accouchement".

On comprend qu'un tel dispositif 1 procure une surveillance temporelle et spatiale efficace et fiable. Naturellement, une variante simplifiée du dispositif 1 consiste à remplacer le dispositif émetteur par une simple sonnerie ou toute autre alarme lumineuse directement attachée à la vache.

Complémentairement, le dispositif 1 est alimenté de façon autonome par un jeu de piles ou de batterie rechargée par exemple au moyen de classiques cellules photovoltaïques.

La présente invention est particulièrement intéressante pour la surveillance de grands troupeaux.

## Revendications

1. Dispositif pour le signalement du vêlage imminent d'une vache en détectant le relevage prolongé de sa queue, comprenant un détecteur de position angulaire temporisé (3, 5, 6) déclenchant un système associé d'alarme autonome (7, 8), caractérisé en ce que le détecteur de position angulaire (3, 5), le relais temporisateur (6) et le système d'alarme (7, 8) sont fixés sur un pince-queue (2), lequel peut être disposé de préférence juste à la naissance de ladite queue.

2. Dispositif suivant la revendication 1 caractérisé en ce que le détecteur de position angulaire (5) est de préférence un contacteur à mercure, à billes ou à lamelles dont la fermeture des contacts produite par un basculement de la queue n'est prise en compte qu'après que la queue soit restée dans cette position un temps prédéterminé grâce à une classique temporisation (6) de préférence électronique.

3. Dispositif suivant l'une quelconque des revendications précédentes caractérisé en ce que le système associé d'alarme (7) est un émetteur radio (8) alimenté de façon autonome par tout moyen connu, transmettant un signal codé à une station réceptrice fixe (9) ou mobile, ou pouvant être relayée par un système autonome et portable, permettant à un surveillant d'être alerté à temps d'un prochain vêlage.

## Claims

1. Device for indicating that a cow is on the point of calving by the detection of the prolonged lifting of its tail, including a time delay angular position detector (3, 5, 6) setting off an associated autonomous alarm detector (7, 8) characterized in that the angular position detector (3, 5) , the delay relay (6) and the alarm system (7, 8) are fixed onto a tail clamp (2) which can be preferably fixed exactly at the base of the said tail.

2. Device in accordance with claim 1 characterized in that the angular position detector (5) is preferably a mercury, ball or blade contactor in which the closing of the contacts caused by the tipping up of the tail is not taken into account until after the tail has remained in this position for a predetermined time thanks to a classical time delay system (6), preferably electronic.

3. Device in accordance with any of the previous claims characterized in that the associated alarm system (7) is a radio emitter (8) supplied autonomously from any known means, transmitting a coded signal to a fixed or mobile receiving station (9) or that can be relayed by an autonomous and portable system enabling a supervisor to be alerted in time that calving is about to take place.

## Patentansprüche

1. Vorrichtung zur Anzeige des bevorstehenden Kalbens einer Kuh durch Detektion des zeitlich ausgedehnten Aufstellens ihres Schwanzes, wobei die Vorrichtung einen Winkelpositionsdetektor mit Verzögerung (3,5,6) enthält, der ein zugehöriges selbständiges Alarmsystem (7,8) auslöst, **dadurch gekennzeichnet**, daß der Winkelpositionsdetektor (3,5), das Verzögerungsrelais (6) und das Alarmsystem (7,8) an einer Schwanzklammer (2) befestigt sind, die vorzugsweise am Anfang des Schwanzes anbringbar ist.

2. Vorrichtung nach Anspruch 1, **dadurch gekennzeichnet**, daß der Winkelpositionsdetektor (5) vorzugsweise ein Quecksilber-, Kugel- oder Lamellenkontakt ist, dessen dem der durch ein Aufstellen des Schwanzes bewirkter Kontaktschluß solange nicht ausgewertet wird, bis der Schwanz für eine durch ein übliches, vorzugsweise elektronisches Verzögerungselement vorherbestimmte Zeitspanne in dieser Position verblieben ist.

3. Vorrichtung nach einem der vorhergehenden Ansprüche, **dadurch gekennzeichnet**, daß das zugehörige Alarmsystem (7) ein mit eigenen bekannten Stromversorgungsmitteln gespeister Radiofrequenzsender (8) ist, der ein codiertes Signal an eine feststehende oder mobile Empfangsstation (9) überträgt oder über eine Relaisstation mit einem selbständigen, tragbaren System verbunden sein kann, um eine Überwachungsperson rechtzeitig über das bevorstehende Kalben zu alarmieren.
